# EUROPEAN PATENT APPLICATION

(11) **EP 1 184 447 A1**
(43) Date of publication of application: **06.03.2002**
(21) Application number: 00119099.0
(22) Date of filing: 04.09.2000
(51) Int. Cl.: C11B 9/00, C07C 45/45, C07C 45/00, C07C 49/67

(54) **Composition having organoleptic characteristics of Safranal**

(71) Applicant: Givaudan SA, 1214 Vernier-Genève (CH)
(72) Inventor: Bajgrowicz, Jerzy A., 8053 Zürich (CH); Gygax, Peter, 8117 Fällanden (CH)
(74) Representative: Patentanwälte Schaad, Balass, Menzl & Partner AG

(57) **Abstract**

3,3-dimethyl-1-indanone has the organoleptic characteristics of safranal and can replace the restricted safranal in fragrance and flavor compositions.

## Description

The present invention relates to a composition having organoleptic characteristics of safranal.

Saffron is a precious spice comprising a lot of different ingredients and is characterized by a sweet, spicy and floral odor with a fatty, herbaceous undertone and a slightly bitter taste. An important ingredient which imparts the characteristic spicy, warm odor of saffron is 2,2,6-trimethyl-4,6-cyclohexadiene-1-carbaldehyde (1) (safranal) (Perfume and Flavor Materials of Natural Origin, S. Arctander, Ed., Elizabeth, N.J., 1960).

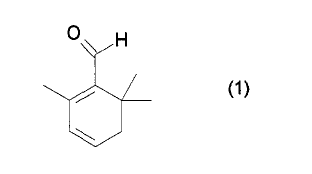

Both saffron and safranal have characteristics which are useful in perfumery. Due to its limited availability and its high price the use saffron tinctures is very limited. Safranal is an extremely valuable and appreciated perfumery ingredient which adds a unique touch to fragrance accords.

Although safranal is a valuable and appreciated flavor ingredient, its use in consumer products should not exceed levels of 0.005%, which is equivalent to 0.025% in a fragrance composition used at 20% in a consumer product. This restriction is based on test results showing sensitization reaction when tested at 0.1% in a human repeated insult patch test.

3,3-dimethyl-1-indanone (2) having the structure 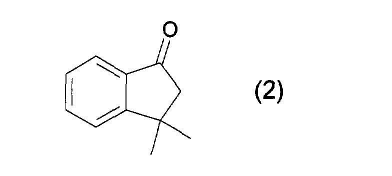 is already described in the literature. Several references exist which either mention its chemical structure or its preparation. US 5,329,049 describes the synthesis of substituted indanones, including 3,3-dimethyl-1-indanone as an example, by reacting aromatic compounds with substituted acrylic acid in liquid hydrogen fluoride. This procedure requires a special apparatus and results beside the desired product in a lot of different by-products which can cause an organoleptic disturbance. The synthesis can be carried out as well as follows:

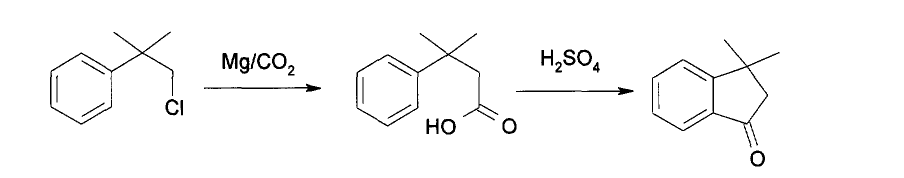

The critical step is the preparation of neophylmagnesiumchloride. Said compound is usually prepared in diethylether (JACS, 149, 97 (1953) or in tetrahydrofurane (J. Organometallic Chemistry, 149, 97 (1978). As diethylether is highly flammable and has a low boiling point, it is not well suited for use in a plant. Tetrahydrofurane (THF) on the other hand has a higher boiling point but is miscible with water which makes the recovery of THF laborious and therefore expensive.

EP 162'465 describes 3,3-dimethyl-1-indanones wherein the phenyl ring is substituted at least once, which are useful in perfumery. US 3'944'679 describes 2-and/or 3-alkylsubstituted indanone compounds having a coumarin-like aroma being useful to impart aroma and flavor to tobacco, foods and drinks. R. Royer et al. (Bull. Soc. Chim. France 1947, 14, 812) describe 3,3-dimethyl-1-indanone to be a liquid with a strong, pleasant solvent-like odor which is not perceptibly pungent.

It is an object of the present invention to provide a fragrance ingredient which has the organoleptic characteristics of safranal and can replace the restricted safranal in fragrance and flavor compositions.

Further it is an object of the present invention to provide an improved method for preparing 3,3-dimethyl-1-indanone.

Surprisingly it was found that 3,3-dimethyl-1-indanone has organoleptic characteristics which resembles very much the complex odor of safranal. Considering the chemical structure and the description of the substance in the literature a person skilled in the art would assume that the substance according to the present invention exhibit an aromatic, solvent-like odor and not a spicy , safranal and leathery odor and taste. Further it was found that 3,3-dimethyl-1-indanone exhibits apart from the spicy, safranal and leathery odor and taste, myrrh, ionone and fruity aspects which is a highly desirable odor and taste combination. Furthermore, it was found that 3,3-dimethyl-1-indanone can ideally substitute safranal in perfumery and flavor applications, and therefore offers an outstanding alternative for the use-limited safranal.

3,3-dimethyl-1-indanone may be used alone or in combination with numerous fragrance and flavor ingredients of natural and/or synthetic origin. The range of the natural fragrances and flavors ingredients includes in addition to highly volatile, also moderately and only slightly volatile components. The synthetic fragrance and flavor ingredient embrace representatives from practically all classes of fragrance and flavor substances. The following list comprises examples of known fragrance and flavor substances which may be combined with the compound of the invention:

### Natural products, e.g.:

tree moss absolute, basil oil, citrus fruit oils (such as bergamot oil, mandarin oil, etc.), myrtle oil, palmarosa oil, patchouli oil, petitgrain oil, wormwood oil, lavender oil, rose oil, jasmin oil, ylang-ylang oil.

### Alcohols, e.g.:

farnesol, geraniol, linalool, nerol, phenylethyl alcohol, rhodinol, cinnamic alcohol, (Z)-hex-3-en-1-ol, menthol, α-terpineol.

### Aldehydes/Ketones, e.g.:

citral, α-hexyl cinnamaldehyde, Lilial® , methylionone, verbenone, nootkatone, geranylacetone.

### Esters / Acetals, e.g.:

allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, Hedione® , styralyl propionate, vetivenyl acetate, benzyl acetate, geranyl acetate, phenyl-acetaldehyde dimethylacetal.

### Lactones, e.g.:

### γ-undecalactone, δ-decalactone, pentadecanolide, 12-oxahexadecanolide.

Further components often used in perfumery: indole, p-mentha-8-thiol-3-one, methyleugenol, eugenol, anethol, or other ingredients described e.g. in "Perfume and Flavor Chemicals", S. Arctander, Ed., Vol. I & II, Allured Publishing Corporation, Carol Stream, USA, 1994 and in "Perfume and Flavor Materials of Natural Origin", S. Arctander, Ed., Elizabeth, N.J., 1960.

The spicy and safranal notes make 3,3-dimethyl-1-indanone an especially valuable ingredient for spicy accords. Furthermore, it blends well with many other spice notes such as nutmeg, clove, pimento, bay, white spice and all spice. The tobacco, leathery character of 3,3-dimethyl-1-indanone enables its use in all "Fougère" or "leathery Fougère" accords for masculine perfumery. It can modify woody accords (cedar, vetyver, sandalwood) or even ambery accords, where it behaves almost as quinoleine or Cistus Labdanum. The fruity aspect of 3,3-dimethyl-1-indanone is of particular interest in apple/pear accords where it enhances aspects or warmth. Due to the ionone-like nuances 3,3-dimethyl-1-indanone blends well with all the lilac, mimosa, acacia and lindenblossom flower notes.

The percentage in which 3,3-dimethyl-1-indanone of the invention is used in a composition may vary within wide limits ranging from 0.1% per weight in mass market products (e.g. cleaning compositions, deodorant, etc.) up to 10% per weight in alcoholic extracts for fine perfumery.

Due to the excellent organoleptic characteristics and its application qualities, 3,3-dimethyl-1-indanone represents alone or in combination with other fragrance and flavor ingredients a valuable fragrance and flavor ingredient for use in any field of fine and functionally perfumery such as perfumes, household products, laundry products, body care products, cosmetics, as well as ingredient for food, drinks and tobacco.

3,3-Dimethylindanone was prepared by an acid catalyzed cyclisation of phenylisobutyric acid, which for its part was prepared by reaction of neophylchloride with magnesium followed by carbon dioxyde. Running the preparation of neophylmagnesiumchloride in MTBE in the presence of about 1/3 equivalent of THF resulted, after quenching with carbon dioxide in mixtures of 2,5-dimethyl-2,5-diphenylhexane and phenylisobutyric acid. If, for example, the neophylchloride was added over a period of 50 minutes to the magnesium, followed by 2½ h of reflux before the addition of carbon dioxide, the ratio of 2,5-dimethyl-2,5-diphenyl-hexane to phenylisobutyric was 54:46. This is in contrast to reactions run under the same conditions in either ether or THF as sole solvent, where practically no 2,5-dimethyl-2,5-diphenyl-hexane is observed.

Surprisingly it was then found that the formation of 2,5-dimethyl-2,5-diphenyl-hexane could be suppressed almost completely by a slow addition of neophylchloride to a magnesium/MTBE suspension in the presence of about 1/3 equivalent of THF. Phenylisobutyric acid can be isolated with yields around 90% when the neophylchloride is added over a period of 5-6h to the magnesium/MTBE/THF suspension.

The invention will be further described, by way of illustration, in the following examples.

### Example 1:

### Preparation of 3,3-Dimethyl-1-indanone

### 1a) 3-Methyl-3-phenyl-butanoic acid (method a)

Magnesium (36.9 g, 1.52 mol, 50 - 150 mesh), neophylchloride (2.0 g, 0.01 mol) and tetrahydrofurane (40 ml) were placed in a flask under an atmosphere of argon. The Grignard reaction was then started with 1,2-dibromoethane (0.2 ml) and tert.-butyl methyl ether (MTBE, 50 ml) was subsequently added while maintaining reflux. Neophylchloride (258 g, 1.53 mol) dissolved in MTBE (400 ml) was then added at 48° C over a period of 5.5 h. After 1.7 h stirring at the same temperature, the reaction mixture was diluted with MTBE (500 ml). Dried CO₂ was then introduced above the surface at a temperature of 35° C (ice cooling) for 18 min and directly into the solution for 5 min. The mixture was then acidified with HCl 16% and the organic phase was extracted with NaOH 32% (160 ml). The alkaline solution was first washed with MTBE and then again acidified with HCl 16%. Extraction with MTBE and evaporation of the solvent gave 3-Methyl-3-phenyl-butanoic acid (247.2 g).
¹H-NMR (CDCl₃) : 10.50-11.00 (broad, 1H), 7.19-7.37(m, 5H), 2.64 (s, 2H), 1.46 (s, 6H).

### 1b) 3-Methyl-3-phenyl-butanoic acid (method b)

Magnesium (4,86 g, 0.2 mol, 50 - 150 mesh), neophylchloride (0.2 g) and tetrahydrofurane(10 ml) were placed in a flask under an atmosphere of argon. The Grignard reaction was then started with 1,2-dibromoethane (0.2 ml). Neophylchloride (35,47 g, 0.2 mol) dissolved in MTBE (50 ml) was then added at reflux over a period of 50 minutes. After stirring for 2 ½ h at the same temperature, the reaction mixture was diluted with MTBE and dried CO₂ was introduced above the surface at a temperature of 35° C (ice cooling). The mixture was then acidified with HCl and extracted with MTBE. GC revealed the presence of 2,5-dimethyl-2,5-diphenyl-hexane and phenylisobutyric in a ratio of 54:46.

### 2) 3,3-Dimethyl-1-indanone

3-Methyl-3-phenyl-butanoic acid (247.2 g, 1.39 mol) was placed in a flask and warmed to 130° C. Sulfuric acid 95% (180 ml) was then added over 4 min and the mixture was stirred at 130° C for 1h, after which more sulfuric acid (10 ml) was added. After stirring for 45 min, the dark solution was poured onto ice (500 g) and extracted with hexane (200 ml). The organic phase was washed with NaHCO₃ (50 ml), dried (MgSO₄) and evaporated. The remaining oil was distilled *in vacuo* (78°_{0,065 Torr}) to give 3,3-dimethyl-1-indanone (157.5 g).
¹H-NMR (CDCl₃): 7.45-7.75 (m, 3H), 7.36 (t, 1H), 2.59 (s, 2H), 1.42 (s, 6H).

### Example 2:

| Pear composition for shampoo/shower gel | | |
|---|---|---|
| Ingredients | Parts per weight | |
| | (+) | (-) |
| Benzyl acetate | 35 | 35 |
| Citronellyl acetate | 35 | 35 |
| cis-3-Hexenyl acetate | 5 | 5 |
| Hexyl acetate | 175 | 175 |
| Prenyl acetate | 5 | 5 |
| Agrumex® ¹⁾ | 75 | 75 |
| Ambrettolide® ²⁾ | 20 | 20 |
| Cyclal C® ²⁾ | 10 | 10 |
| Allyl cyclohexyl-propionate | 10 | 10 |
| Ethyl decadienoate | 1 | 1 |
| Givescone® ²⁾ | 15 | 15 |
| Phenoxyethyl isobutyrate | 314 | 304 |
| Lilial® ²⁾ | 60 | 60 |
| Nectaryl® ²⁾ | 30 | 30 |
| Verdyl propionate® ²⁾ | 150 | 150 |
| Hexyl salicylate | 60 | 60 |
| 3,3-Dimethyl-1-indanone | 10 | 0 |
| Total | 1000 | 1000 |

| | | |
|---|---|---|
| ¹⁾ origin: International Flavors & Fragrances, USA | | |
| ²⁾ origin: Givaudan Roure (International) SA, Vernier, Switzerland | | |

In this classical pear accord for a shampoo / shower gel, the use of 3,3-dimethyl-1-indanone is particularly noticeable: the compound brings an extraordinary volume and warmth to the fragrance, the pear aspect becomes much more juicy and natural, with a sweet cooked undertone. The harshness of the pear top notes is smoothened by the safranal/woody aspects of 3,3-dimethyl-1-indanone.

### Example 3:

| Coffee composition for a fine fragrance | | |
|---|---|---|
| Ingredients | Parts per weight | |
| | (+) | (-) |
| Acetyl-pyrazine | 0.5 | 0.5 |
| Civettone¹⁾ | 2.0 | 2.0 |
| Corylone® ²⁾ | 3.5 | 3.5 |
| Damascenone¹⁾ | 1.0 | 1.0 |
| Dipropylene glycol | 83.8 | 85.8 |
| Ethyl dimethyl pyrazine at 1%³⁾ | 1.0 | 1.0 |
| Fenugreek abs. at 10%⁴⁾ | 0.5 | 0.5 |
| Furfuryl mercaptan at 1%³⁾ | 0.7 | 0.7 |
| Guaiacol | 0.5 | 0.5 |
| Maltol | 1.0 | 1.0 |
| 2-Methylpyrazine at 1%³⁾ | 1.0 | 1.0 |
| Isoeugenol acetate | 2.0 | 2.0 |
| Vanilline | 1.0 | 1.0 |
| 3,3-Dimethyl-1-indanone | 2.0 | 0 |
| Total | 100 | 100 |

| | | |
|---|---|---|
| ¹⁾ origin: Firmenich S.A., Switzerland | | |
| ²⁾ origin: Givaudan Roure (International) SA, Vernier, Switzerland | | |
| ³⁾ in triethyl citrate | | |
| ⁴⁾ in PG | | |

In this coffee accord 3,3-dimethyl-1-indanone with its warm, spicy/saffran note gives warmth, richness and volume to the fragrance.

The systematic names of the individual components listed in the examples above are listed in standard works, e.g. "Perfume and Flavor Chemicals", S. Arctander, Ed., Vol. I and II, Allured Publishing Corporation, Carol Stream, USA, 1994.

## Claims

1. A composition having organoleptic characteristics of safranal comprising 3,3-dimethyl-1-indanone.

2. Composition according to claim 1 further comprising a fragrance ingredient.

3. Composition according to any of the preceding claims further comprising a compound with a spicy accord.

4. Composition according to any of the preceding claims further comprising a compound with a leathery accord.

5. Composition according to any of the preceding claims further comprising a compound with a fougère accord.

6. Composition according to any of the preceding claims further comprising a compound with a leathery fougère accord.

7. Composition according to any of the preceding claims further comprising a compound with a woody accord.

8. Composition according to any of the preceding claims further comprising a compound with an ambery accord.

9. Composition according to any of the preceding claims further comprising a compound with a fruity accord.

10. Composition according to any of the preceding claims further comprising a compound with a flower note.

11. Consumer products comprising a composition according to any of the preceding claims.

12. Household product comprising a composition according to any of the preceding claims.

13. Body care product comprising a composition according to any of the preceding claims.

14. Food and beverage products comprising a composition according to any of the preceding claims.

15. Tobacco products comprising a composition according to any of the preceding claims.

16. Use of 3,3-dimethyl-1-indanone as fragrance ingredient.

17. Use of 3,3-dimethyl-1-indanone as flavor ingredient.

18. A method for preparing 3,3-dimethyl-1-indanone by reaction of magnesium and neophenylchloride in a solvent system consisting of MTBE and THF, wherein neophenylchloride dissolved in a MTBE is added in a time range of 3 to 6 h, treating the intermediate with CO₂ resulting in phenylisobutyric acid which is converted to the 3,3-dimethyl-1-indanon by acid catalyzed cyclization.
